# EUROPEAN PATENT SPECIFICATION

(11) **EP 1 545 323 B1**
(45) Date of publication and mention of the grant of the patent: **12.04.2006**
(21) Application number: 02765181.9
(22) Date of filing: 03.09.2002
(51) Int. Cl.: A61B 17/02, A61B 17/34

(54) **DEVICE FOR STABILISING AND/OR POSITIONING A MEDICAL TOOL IN A BODY CAVITY**
VORRICHTUNG ZUR STABILISIERUNG UND/OR ZUM POSITIONIEREN EINES MEDIZINISCHEN WERKZEUGES IN EINE KÖRPERHÖHLE
DISPOSITIF DESTINE A STABILISER ET/OU A POSITIONNER UN INSTRUMENT MEDICAL DANS UNE CAVITE CORPORELLE

(43) Date of publication of application: 29.06.2005
(73) Proprietor: UNIVERSITE DE NEUCHATEL, 2000 Neuchatel (CH)
(72) Inventor: STAUFER, Urs, CH-2000 Neuchâtel (CH); IMER, Rapha¬l, CH-2072 St. Blaise (CH)
(74) Representative: Blum, Rudolf Emil
(86) International application number: PCT/IB2002/003602
(87) International publication number: WO 2004/021892

(56) References cited:
- WO-A-99/40971
- DE-A- 4 104 586
- US-A- 5 601 590
- US-A- 5 925 058
- US-A- 6 068 611
- US-A1- 2002 095 160

## Description

### Technical Field

The present invention relates to a medical instrument according to the preamble of claim 1. Such an instrument is e.g. described in DE 41 04 586 A1 and US 2002/009516

### Background Art

The current evolution in nanotechnology enables observations and measurements of sub-micron and nanosize objects by means of scanning probes. This scale range is attractive for analyzing diseased human tissues.

During a classical surgery session, the stabilization of the tool is entirely relaying on the support by the surgeon's hand. Various types of mechanisms outside the body have been designed to block the instrument in a desired position. These mechanisms reduce unwanted movement of the tool's tip. However, the achieved stabilization is likely to be insufficient for nanoscale measurements.

Therefore, there is a need for a device allowing a sufficiently precise stabilisation and/or positioning of a medical tool in a body cavity so that nanoscale measurements on human tissues can be performed.

### Disclosure of the Invention

Hence, it is a general object of the present invention to provide a medical instrument for stabilising and/or positioning a medical tool in a body cavity. Said instrument comprises an elongated tubular structure with an end for insertion in a body cavity, at least one inflatable balloon connected to at least one capillary tube and inflatable by pressing a liquid and/or gas through said capillary tube and a means for receiving a medical tool. Said at least one balloon is arranged at a distance from said medical tool.

The instrument further comprises an atomic force microscope (AFM) and a means for receiving the same, which allows to perform nanoscale measurements in the body cavity.

The term "body cavity" as used herein encompasses not only any hollow space within a body or any of its organs but as well the space in joints or the lumen of blood and lymph vessels.

In a preferred embodiment of the present invention said elongated tubular structure further comprises at least one surface opening and said at least one inflatable balloon is located at said at least one surface opening, preferably at least two surface openings and at least two inflatable balloons located at said surface openings, more preferably at least four surface openings and at least four inflatable balloons located at said surface openings.

In a further preferred embodiment said surface openings are uniformly distributed along a circumference of said elongated tubular structure and the centres of all said surface openings are in the same distant from the end of the elongated tubular structure to be inserted in the body cavity.

In another preferred embodiment the means for receiving the AFM is a recess having a polygonal profile.

In a further preferred embodiment said elongated tubular structure further comprises a means for forcing the balloons to expand outside said elongated tubular structure when the balloons are inflated. Preferably, said means for forcing the balloons to expand outside said elongated tubular structure is a ring.

In a further preferred embodiment said capillary tubes connected to said inflatable balloons are fixed to said means for forcing the inflated balloons outside said elongated tubular structure.

The present invention is suitable for stabilising and/or positioning the AFM (Atomic force microscope).

The present invention allows a precise stabilisation of the AFM tip which is very sensitive to vibrations and allows to perform measurements in a sub-micron and nanosize scale range in the human body e.g. on diseased human tissue. The data of said measurements of diseased human tissue can be used for diagnostic purposes and can help to select a suitable therapy method.

The careful regulation of the relative pressure in the balloons allows for a positioning the medical tool relative to the tissue to be examined. In fact, the inflation or the deflation of a balloon approaches or withdraws the medical tool from the sample. A combination of e.g. four balloons increases appreciably the precision of the technique. When the positioning is finished, the inflating system can be deactivated and so it does not interfere with the measurement.

### Brief Description of the Drawings

The invention will be better understood and objects other than those set forth above will become apparent when consideration is given to the following detailed description thereof. Such description makes reference to the annexed drawings, wherein:
Fig. 1a shows a top plan view of the device 20 inside of a knee. Inflated balloons 4 exert a small pressure on the kneecap 9 and on the tibia bone 10;
Fig 1b shows an enlarged part of Fig. 1a;
Fig. 2 shows a perspective view of the device 20, showing the surface openings 2 in the elongated tubular structure 1 and flat balloons 3;
Fig. 3a shows a cross-sectional view of the device 20 with flat 3 balloons;
Fig 3b shows a cross-sectional view of the device 20 with inflated balloons 4;
Fig. 4a shows a cross-sectional view of the device 20, using differential pressure in the inflatable balloons 4 for positioning the AFM relative to the human tissue that is examined and
Fig. 4b shows a cross-sectional view of the device 20, using differential pressure in the inflatable balloons 4 for positioning the AFM relative to the human tissue that is examined.

### Modes for Carrying Out the Invention

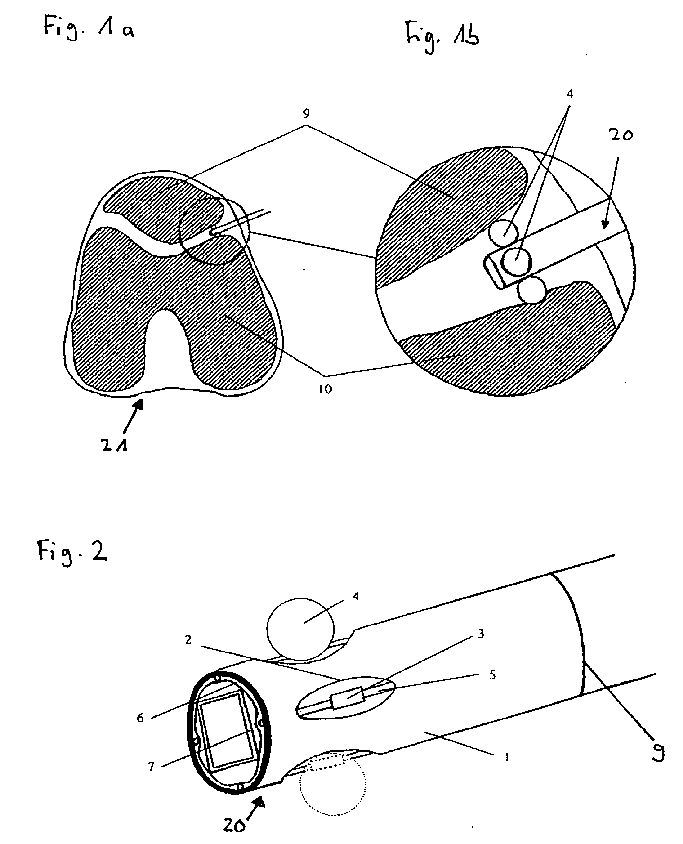

Fig.1a and b show an exemplary use of a device 20 for stabilising and/or positioning the AFM in the knee 21 of a subject in order to perform in vivo AFM measurements.

As during a state of the art arthroscopy, several little incisions are made in the patient's knee, depending on the required tools. Through a first opening, the physiological liquid is introduced. Through a second one, an optical fibre based microscope, the arthroscope is introduced, which allows real-time and colour imaging of the inside of the knee. The device 20 with an AFM can be introduced through a further incision in the same way as standard minimal invasive tools are employed.

After its insertion, the inventive device 20 can be guided to the right position with the help of the arthroscope. Then, the stabilization is performed by inflating the balloons 3,4 of the device 20. Balloons' expansion exert a small pressure on the bordering tissues, solidly fixing the tool in the knee.

Balloons can be inflated by gas or liquid. For two main reasons the use of physiological liquid is preferred:

The first one is a safety reason. Physiological liquid, a mixture of water and different salts, is widely used in medicine. If the balloons of the inventive device with a medical tool such as e.g. a nanoinstrument are activated with the same liquid, no harm is expected in case of a leak.

The second reason is that vibrations are more effectively damped in a liquid. Therefore using physiological solution to inflate balloons has the additional advantage of absorbing small movements and vibrations around of the tool. The feeding of the liquid and/or gas through the capillary tubes 5 to inflate the balloons 3,4 can e.g. be done by a pump.

Fig. 2 shows a perspective view of an exemplary embodiment of the device 20 of the present invention.

In said embodiment the inventive device 20 comprises an elongated tubular structure 1 with oval surface openings 2, inflatable balloons 3,4 connected to capillary tubes 5 and located in the surface openings 2, a means 6 for forcing the balloons to expand outside the elongated tubular structure 1, a means 7 for receiving a medical tool and a connecting means 9.

The means 7 for receiving the AFM can be any structure allowing a secure fixation of the medical tool to the elongated tubular structure 1. In a preferred embodiment said structure is a recess with a polygonal profile.

The capillary tubes 5 connected to the inflatable balloons 3,4 can be fixed to the inside of the elongated tubular structure 1 in such a way that the balloons 3,4 are forced to expand outside the surface openings 2 of the tubular elongated structure 1.

The size, shape, number and arrangement of the surface openings 2 on the elongated tubular structure 1 can vary. The optimal arrangement of the surface openings 2 for a specific body cavity depends on the shape and size of the body cavity. To achieve a good stabilisation and/or positioning of the AFM in a cavity with an uneven shape, the device 20 comprises preferably at least four surface openings 2. The centres of said surface openings 2 can e.g. be uniformly distributed along a circumference of the elongated tubular structure 1. When more than four surface openings 2 are present on the tubular structure 1, said surface openings can e.g. be arranged in two planes and be uniformly distributed along a circumference of the elongated tubular structure 1.

The size and shape of the surface openings 2 has to be large enough so that the inflated balloons 4 can pass through said openings 2 and get located on the surface of the elongated tubular structure 1. It is important that the surface openings 2 are arranged on the elongated tubular structure 1 in such a manner that they are inside the body cavity when the device 20 is inserted in the body cavity. In an exemplary embodiment of the inventive device the balloons 3,4 can be located outside the elongated tubular structure 1 and be connected to the capillary tubes 5 through the wall of the elongated tubular structure 1.

The elongated tubular structure 1 can comprise a first part which is for insertion in the body cavity and a second part which is for handling the device 20 outside the body wherein said two parts are connected by a connecting means 9. Such a connecting means 9 can e.g. be a bayonet joint or a thread. Said connecting means 9 allows the removal of the part of the elongated tubular structure 1 which is outside the body after the stabilisation and/or positioning of the AFM in the body cavity has been achieved and said removal can further improve the stabilisation and/or positioning of the medical tool in the body cavity.

Figure 3a shows a cross-sectional view of a device 20, with surface openings 2 and deflated balloons 3 connected to capillary tubes 5. In the shown embodiment the means 6 for forcing the inflated balloons outside the elongated tubular structure 1 is a ring which runs along the inner circumference of the elongated tubular structure 1. The person skilled in the art knows other suitable embodiments of structures for forcing the inflated balloons 4 outside the elongated tubular structure 1.

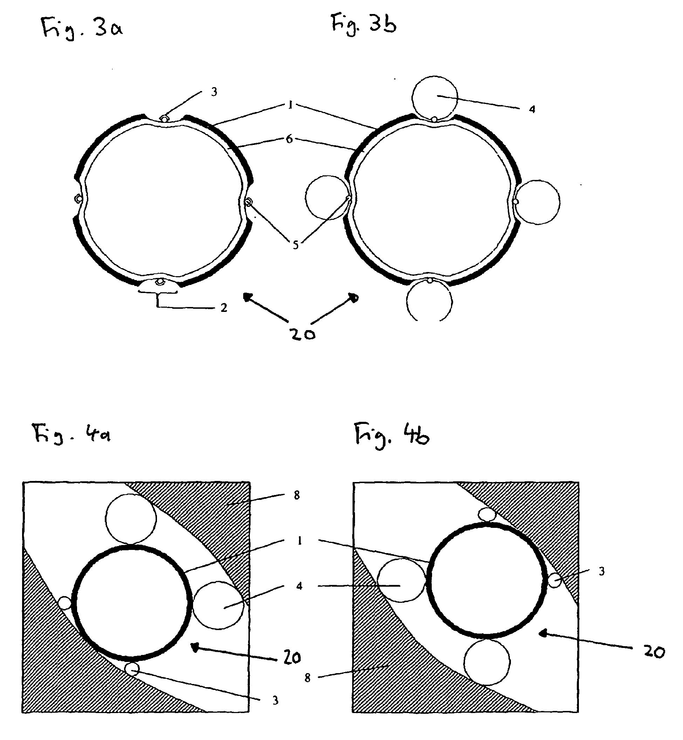

Figure 3b shows a cross-sectional view of a device 20, with inflated balloons 4. The balloons 3,4 can comprise on their surface protuberances to achieve a good stabilisation on the inner surface of the body cavity or tissue.

Figure 4a shows a cross-sectional view of a device 20 applied in an exemplary body cavity. The exemplary embodiment comprises four balloons 3,4 which have been inflated using different pressure.

Figure 4b shows a cross-sectional view of a device 20 applied in a further exemplary body cavity. The exemplary embodiment comprises four balloons 3,4 which have been inflated using different pressure.

The embodiments of Fig. 4a and 4b show that the device 20 of the present invention allows for a positioning of the AFM in body cavities with variable shapes. Depending on the shape of the cavity different pressures can be used to inflate the single balloons 3,4 in order to achieve a stable position of the AFM in the body cavity. The inflatable balloons 3,4 can comprise on their surface protrusions in order to optimise the attachment of the inflated balloons 4 to e.g. a rough surface of a bone.

Materials that can be used for the construction of the inventive device are known to a person skilled in the art. The elongated tubular structure can e.g. be made of any biocompatible material such as e.g. stainless steel. The capillary tubes 5 can e.g. be made of the material used to produce heart catheters.

While there are shown and described presently preferred embodiments of the invention, it is to be distinctly understood that the invention is not limited thereto but may be otherwise variously embodied and practised within the scope of the following claims.

## Claims

1. A medical instrument comprising
an elongated tubular structure (1) with an end for insertion in the body cavity and
at least one inflatable balloon (3,4) connected to at least one capillary tube (5) and inflatable by pressing a fluid and/or gas into said tube,
said instrument further being **characterized by**
an atomic force microscope and
a means (7) for receiving said atomic force microscope.

2. The instrument of claim 1, wherein said at least one balloon (3,4) is arranged at a distance from said atomic force microscope.

3. The instrument of any of claims 1 or 2, wherein said elongated tubular structure (1) further comprises at least one surface opening (2) and said at least one inflatable balloon (3,4) is located at said at least one surface opening (2), preferably at least two surface openings (2) and at least two inflatable balloons (3,4) located at said surface openings (2), more preferably at least four surface openings (2) and at least four inflatable balloons (3,4) located at said surface openings (2).

4. The instrument of claim 3, wherein said surface openings (2) are uniformly distributed along a circumference of said elongated tubular structure (1) and the centres of all surface openings (2) are at equal distant from the end of the elongated tubular structure (1) to be inserted in the body cavity.

5. The instrument of any of claims 1 to 4, wherein said means (7) for receiving said atomic force microscope is a recess having a polygonal profile.

6. The instrument of any of claims 1 to 5, wherein said elongated tubular structure (1) further comprises a means (6) for forcing the balloons to expand outside said elongated tubular structure (1).

7. The instrument of claim 6, wherein said means (6) for forcing the balloons to expand outside said elongated tubular structure (1) is a ring.

8. The instrument of any of claims 6 or 7, wherein said capillary tubes (5) are fixed to said means (6) for forcing the balloons to expand outside said elongated tubular structure (1).

9. The instrument of any of the claims 1 to 8 comprising several balloons, wherein said balloons are inflatable by different pressures.

10. The instrument of any of claims 1 to 8, wherein said elongated tubular structure (1) comprises a first part and a second part which are connected by a connecting means (9).

## Patentansprüche

1. Ein medizinisches Instrument einschliesslich
einer gestreckten röhrenförmigen Struktur (1) mit einem Ende zum Einführen in die Körperöffnung und
mindestens einem aufblasbaren Ballon (3, 4), der mit mindestens einem Kapillarrohr (5) verbunden und durch das Einpressen einer Flüssigkeit und/oder eines Gases in das genannte Rohr aufblasbar ist,
wobei das genannte Instrument des Weiteren **gekennzeichnet ist durch**
ein Rasterkraftmikroskop und
einem Mittel (7) zur Aufnahme des genannten Rasterkraftmikroskops.

2. Das Instrument nach Anspruch 1, wobei der genannte mindestens eine Ballon (3, 4) vom genannten Rasterkraftmikroskop entfernt angeordnet ist.

3. Das Instrument nach einem der Ansprüche 1 oder 2, wobei die genannte röhrenförmige Struktur (1) des weiteren mindestens eine Oberflächenöffnung (2) umfasst und der genannte mindestens eine aufblasbare Ballon (3, 4) bei der genannten mindestens einen Oberflächenöffnung (2) angeordnet ist, vorzugsweise mindestens zwei Oberflächenöffnungen (2) und mindestens zwei, bei den Oberflächenöffnungen (2) angeordnete, aufblasbare Ballone (3, 4), und noch bevorzugter mindestens vier Oberflächenöffnungen (2) und mindestens vier, bei den Oberflächenöffnungen (2) angeordnete, aufblasbare Ballone (3, 4).

4. Das Instrument nach Anspruch 3, wobei die genannten Oberflächenöffnungen (2) gleichmässig entlang einem Umfang der genannten gestreckten röhrenförmigen Struktur (1) verteilt sind und die Zentren aller Oberflächenöffnungen (2) den gleichen Abstand vom Ende der gestreckten röhrenförmigen Struktur (1) haben, die in die Körperöffnung einzuführen ist.

5. Das Instrument nach einem der Ansprüche 1 bis 4, wobei das Mittel (7) zur Aufnahme des genannten Rasterkraftmikroskops eine Aussparung mit einem polygonalen Profil ist.

6. Das Instrument nach einem der Ansprüche 1 bis 5, wobei die genannte gestreckte röhrenförmige Struktur (1) des Weiteren ein Mittel (6) umfasst, das die Ballone zwingt, sich ausserhalb der genannten gestreckten röhrenförmigen Struktur (1) auszudehnen.

7. Das Instrument nach Anspruch 6, wobei das genannte Mittel (6), das die Ballone zwingt, sich ausserhalb der genannten gestreckten röhrenförmigen Struktur (1) auszudehnen, ein Ring ist.

8. Das Instrument nach einem der Ansprüche 6 oder 7, wobei die genannten Kapillarrohre (5) an dem Mittel (6), das die Ballone zwingt, sich ausserhalb der genannten gestreckten röhrenförmigen Struktur (1) auszudehnen, befestigt sind.

9. Das Instrument nach einem der Ansprüche 1 bis 8 einschliesslich mehreren Ballonen, wobei die genannten Ballone durch verschiedene Drücke aufblasbar sind.

10. Das Instrument nach einem der Ansprüche 1 bis 8, wobei die genannte gestreckte röhrenförmige Struktur (1) einen ersten Teil und einen zweiten Teil umfasst, die mittels eines Verbindungsmittels (9) verbunden sind.

## Revendications

1. Instrument médical comprenant :
une structure tubulaire oblongue ayant une extrémité d'insertion (1) dans la cavité du corps et
au moins un ballon (3, 4) gonflable communiquant avec au moins un tube (5) capillaire et pouvant être gonflé en refoulant un fluide et/ou gaz dans le tube,
l'instrument étant **caractérisé en outre par** un microscope de force atomique et
un moyen (7) de réception du microscope de force atomique.

2. Instrument suivant la revendication 1, dans lequel au moins un ballon (3, 4) est disposé à distance du microscope de force atomique.

3. Instrument suivant l'une des revendications 1 ou 2, dans lequel la structure (1) tubulaire oblongue comporte en outre au moins une ouverture (2) en surface et le au moins un ballon (3, 4) gonflable est placé sur la au moins une ouverture (2) en surface, de préférence sur au moins deux ouvertures (2) en surface et au moins deux ballons (3,4) sont placés sur les ouvertures (2) en surface, mieux encore sur quatre ouvertures (2) en surface et au moins quatre ballons (3,4) gonflables sont placés sur les ouvertures (2) en surface.

4. Instrument suivant la revendication 3 dans lequel les ouvertures (2) en surface sont réparties uniformément sur une circonférence de la structure tubulaire oblongue (1) et les centres de toutes les ouvertures (2) en surface sont équidistants de l'extrémité de la structure (1) tubulaire oblongue à insérer dans la cavité du corps.

5. Instrument suivant l'une quelconque des revendications 1 à 4, dans lequel le moyen (7) de réception du microscope de force atomique est un chambrage ayant un profil polygonal.

6. Instrument suivant l'une quelconque des revendications 1 à 5, dans lequel la structure (1) tubulaire oblongue comprend un moyen (6) pour forcer les ballons à s'agrandir à l'extérieur de la structure (1) tubulaire oblongue.

7. Instrument suivant la revendication 6, dans lequel le moyen (6) pour forcer les ballons à s'agrandir à l'extérieur de la structure (1) tubulaire oblongue est un anneau.

8. Instrument suivant l'une des revendications 6 ou 7, dans lequel les tubes (5) capillaires sont fixés au moyen (6) pour forcer les ballons à s'agrandir à l'extérieur de la structure (1) tubulaire oblongue.

9. Instrument suivant l'une quelconque des revendications 1 à 8, comprenant plusieurs ballons, les ballons pouvant être gonflés à des pressions différentes.

10. Instrument suivant l'une quelconque des revendications 1 à 8, dans lequel la structure (1) tubulaire oblongue comprend une première et une deuxième partie qui sont reliées par des moyens (9) de liaison.
